# FASCICULE DE BREVET EUROPEEN

(11) **EP 1 731 195 B1**
(45) Date de publication et mention de la délivrance du brevet: **30.01.2008**
(21) Numéro de dépôt: 06290944.5
(22) Date de dépôt: 09.06.2006
(51) Int. Cl.: A61N 1/368

(54) **Dispositif médical implantable actif AAI/DDD, notamment stimulateur cardiaque, à gestion perfectionnée des commutations de mode en présence d'évènements ventriculaires de nature incertaine**
Aktive implantierbare medizinische Vorrichtung mit AAI/DDD Modusschaltung, insbesondere Herzschrittmacher, mit verbesserter Steuerung der Modusschaltung in Anwesenheit von unsicheren ventrikulären Ereignissen
Active implantable medical device with AAI/DDD mode switching, in particular pacemaker, with improved control of mode switching in the presence of uncertain ventricular events

(30) Priorité: 09.06.2005 FR 0505852
(43) Date de publication de la demande: 13.12.2006
(73) Titulaire: ELA MEDICAL (Société anonyme), 92541 Montrouge (FR)
(72) Inventeur: Amblard, Amel, 92290 Chatenay Malabry (FR)
(74) Mandataire: Dupuis-Latour, Dominique

(56) Documents cités:
- EP-A- 0 875 264
- EP-A- 1 470 836

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, dispositifs "multisite" (triple ou quadruple chambre), défibrillateurs et/ou cardioverteurs, permettant de surveiller l'activité cardiaque et de délivrer au coeur des impulsions électriques de stimulation, de resyn-chronisation, de cardioversion et/ou de défibrillation en cas de trouble du rythme diagnostiqué par l'appareil.

Elle concerne plus particulièrement ceux de ces dispositifs qui comprennent des circuits de stimulation et de détection à la fois sur l'oreillette et sur le ventricule et qui peuvent opérer selon deux modes de fonctionnement, DDD ou AAI (le mode AAI étant en fait un mode DDD modifié par allongement du délai auriculo-ventriculaire). Ces dispositifs peuvent être pourvus d'un mode dénommé "DDD-CAM" ou "AAISafeR" assurant une commutation automatique de mode (CAM) de DDD en AAI et inversement.

Le mode de fonctionnement de base d'un stimulateur DDD/AAI est un mode AAI - ou plus précisément "pseudo-AAI" - avec stimulation auriculaire simple chambre (mode AAI *stricto sensu*) et surveillance de l'activité ventriculaire. Ce mode est en principe maintenu tant que la conduction auriculo-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs auriculo-ventriculaires (BAV) entraînant un trouble temporaire de dépolarisation du ventricule. Dans ce cas, dès lors qu'un certain nombre de conditions sont remplies, le stimulateur bascule automatiquement en mode DDD automatique, avec des paramètres optimisés pour cette situation de BAV temporaire. Après disparition du BAV, et donc rétablissement de la conduction auriculo-ventriculaire, le stimulateur retourne automatiquement au mode AAI dès lors qu'un certain nombre d'autres conditions sont remplies.

Cette commutation entre modes DDD et AAI est notamment décrite dans les EP-A-0 488 904 et EP-A-1 346 750 (ELA Médical).

Le point de départ de l'invention réside dans un certain nombre d'observations effectuées lors d'un suivi clinique de patients appareillés avec des dispositifs DDD/AAI à commutation automatique de mode.

Il est apparu en effet que ces appareils présentent une spécificité insuffisante de la détection des troubles ventriculaires, de sorte que dans certains cas se produisent des commutations inappropriées vers le mode DDD.

En effet, le principe de base d'un tel dispositif est que, lorsqu'il se trouve en mode AAI, toute détection ventriculaire maintient le dispositif en mode AAI (c'est-à-dire inhibe la commutation en mode DDD), sauf lorsque sont vérifiées les conditions permettant de suspecter l'apparition d'un BAV.

Le dispositif considère qu'il y a BAV lorsque sont vérifiés un certain nombre de critères révélant une conduction présente mais retardée (BAV du premier degré), ou que certaines ondes P ne sont plus conduites (BAV du deuxième degré) ou sont totalement bloqués (BAV complet, ou BAV du troisième degré).

Le basculement en mode DDD peut également être déclenché sur diagnostic d'une pause ventriculaire, c'est-à-dire lorsque l'intervalle séparant deux événements ventriculaires excède un délai donné.

La détection d'une dépolarisation ventriculaire spontanée, et le calcul de l'intervalle de temps la séparant du précédent événement auriculaire, sont donc essentiels pour le diagnostic d'un BAV, et par voie de conséquence pour le basculement éventuel en mode DDD.

Or, après délivrance d'une impulsion de stimulation auriculaire, le dispositif applique au circuit de détection ventriculaire une période dite "fenêtre de sécurité", typiquement de 100 ms après la stimulation auriculaire, telle que si une dépolarisation est détectée avant la fin de cette fenêtre, elle est ignorée par le dispositif ; en effet, compte tenu du délai très court séparant cette détection de la stimulation auriculaire antérieure, il peut s'agir de la détection d'un artéfact électrique lié par exemple au temps de récupération de l'amplificateur de détection.

Pour cette raison, dans les dispositifs connus, une telle dépolarisation est généralement ignorée et n'est pas considérée comme la fin (ou le début) d'un cycle ventriculaire, notamment pour la gestion des divers intervalles (intervalle d'échappement, délai ventriculo-auriculaire) et la gestion de la commutation éventuelle du mode AAI vers le mode DDD.

Le EP-A-1 470 836 utilise la détection d'une telle dépolarisation pour identifier une possible extrasystole ventriculaire tardive masquée par la stimulation ventriculaire, et qui pourrait leurrer l'algorithme d'analyse du rythme.

Mais,dans certains cas il peut s'agir d'une réelle activité ventriculaire, le faible délai AV pouvant s'expliquer alors :
- soit par un dysfonctionnement du système, notamment consécutif à un défaut de détection d'un événement auriculaire spontané,
- soit par un véritable asynchronisme auriculo-ventriculaire : allongement pathologique du délai AV, rythme jonctionnel, insuffisance chronotrope avec accélération du rythme ventriculaire, etc.

En l'absence de vérification de la nature réelle de l'événement détecté en fenêtre de sécurité, le dispositif considère, par mesure de sécurité, qu'il y a défaut de conduction auriculo-ventriculaire de sorte que la survenue de plusieurs événements de ce type peut conduire à un basculement vers le mode DDD.

Ainsi, outre la non-détection de l'éventuel trouble ventriculaire à l'origine de cette situation, le fonctionnement du dispositif est perturbé par un faux diagnostic provoquant une commutation inopportune du dispositif en mode DDD.

Bien qu'un fonctionnement en mode DDD n'ait généralement pas de conséquence délétère pour le patient, une telle commutation est inutile et empêche l'expression de la conduction auriculo-ventriculaire spontanée.

Un premier but de la présente invention est de proposer un dispositif qui permette de caractériser les phénomènes de ce type correspondant à une réelle activité ventriculaire et, dans le cas où des commutations vers le mode DDD sont intervenues, de pouvoir identifier ces commutations particulières, notamment pour les présenter ultérieurement au médecin en les distinguant des commutations liées à un trouble avéré de conduction.

Un autre but de invention est de proposer un dispositif qui, outre la caractérisation de ces phénomènes, aménage la commutation automatique de mode pour éviter un basculement intempestif en mode DDD dans les cas particuliers ainsi identifiés.

Le type de dispositif auquel s'applique l'invention est un dispositif de type "AAISafeR" d'un type connu, par exemple selon le EP-A-1 470 836 précité, correspondant au préambule de la revendication 1. Les éléments spécifiques de l'invention sont énoncés dans la partie caractérisante de cette revendication 1. Les sous-revendications visent des formes particulières de mise en oeuvre de l'invention.

On va maintenant décrire un exemple de mise en oeuvre du dispositif de l'invention, en référence aux dessins annexés.

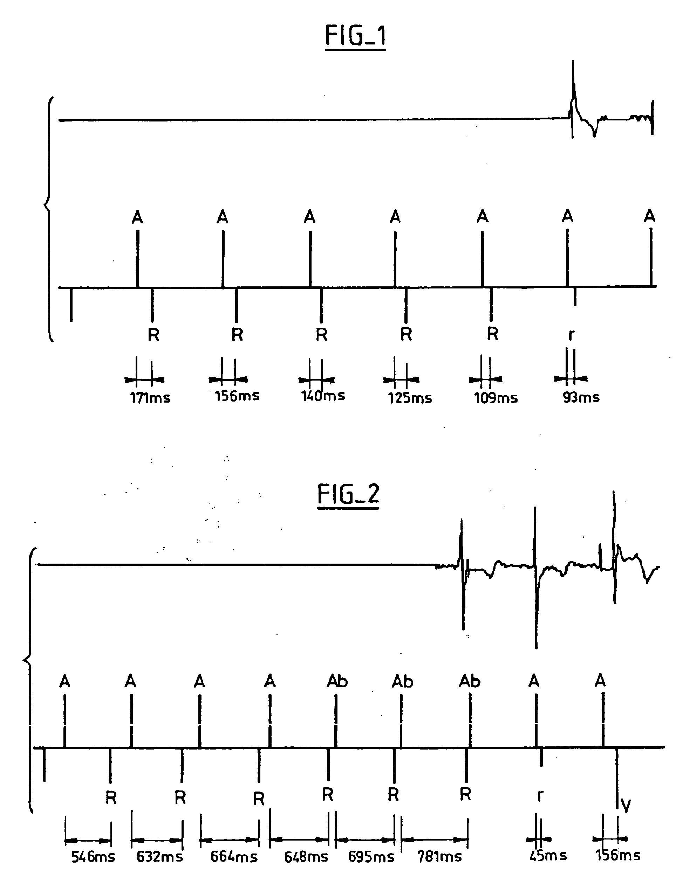

Les figures 1 et 2 sont des chronogrammes montrant deux situations de survenue d'événements ventriculaires particuliers détectés conformément aux enseignements de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony* et *ELA Rhapsody* et les défibrillateurs *Alto* et *Ovatio.*

Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Sur la figure 1, les marqueurs A illustrent la position dans le temps des impulsions de stimulation auriculaire successives, caractéristiques d'un fonctionnement en mode AAI.

En cas de conduction auriculo-ventriculaire normale, c'est-à-dire en l'absence de BAV, le dispositif détecte après chaque stimulation une dépolarisation ventriculaire consécutive R associée.

Après délivrance d'une impulsion de stimulation, le dispositif applique toutefois au circuit de détection ventriculaire une période dite "fenêtre de sécurité" typiquement d'une durée de 100 ms à partir de la stimulation auriculaire.

Cette fenêtre est telle que si une dépolarisation est détectée avant la fin de la fenêtre de sécurité, elle est ignorée par le dispositif, car dans ce cas, compte tenu de sa proximité avec la stimulation auriculaire antérieure, la nature de l'événement détecté est incertaine : il peut notamment s'agir de la détection d'un artéfact électrique lié par exemple au temps de récupération de l'amplificateur de détection du signal ventriculaire, ou encore d'une extrasystole ventriculaire (ESV) isolée, phénomènes dont la prise en compte pourrait perturber le déroulement normal de l'algorithme d'analyse du rythme cardiaque.

Mais dans certains cas particuliers cette détection ventriculaire en fenêtre de sécurité peut être révélatrice d'une véritable activité ventriculaire.

Le délai AR court peut alors s'expliquer soit par un dysfonctionnement du système (défaut de détection auriculaire), soit par un véritable asynchronisme auriculo-ventriculaire : notamment allongement pathologique du délai auriculo-ventriculaire, rythme jonctionnel, ou insuffisance chronotrope avec accélération du ventricule.

Dans les dispositifs de l'art antérieur, par mesure de sécurité, le dispositif considère qu'une stimulation auriculaire A suivie d'une détection ventriculaire R en fenêtre de sécurité est corrélée à un BAV, de sorte que la survenue de plusieurs événements de ce type peut conduire à une commutation en mode DDD.

Selon un premier aspect de l'invention, il est proposé de caractériser les commutations liées à ce phénomène, de manière à leur associer un marqueur spécifique qui puisse être ultérieurement présenté au praticien lors de la lecture de l'enregistrement réalisé par le dispositif, en distinguant ces commutations de celles liées à un trouble avéré de conduction AV.

Pour ce faire, chaque fois que survient une commutation impliquant dans le dernier cycle ventriculaire une stimulation auriculaire suivie d'une détection ventriculaire en fenêtre de sécurité, le dispositif associe à cette commutation un marqueur spécifique indicatif d'une "commutation non liée à un BAV".

De même, tout épisode démarrant par une telle "commutation non liée à un BAV" sera affecté d'un marqueur indicatif d'un "épisode non lié à un BAV". Un "épisode" est une succession de cycles débutant sur une commutation AAI → DDD, et se terminant par exemple après 100 cycles consécutifs en mode DDD sans retour au mode AAI (le dispositif pourra alors forcer le retour au mode AAI dans certaines conditions de manière à permettre à une éventuelle conduction AV de s'exprimer).

Ces commutations et épisodes "non liés à un BAV" sont documentés par des marqueurs particuliers et font l'objet de statistiques spécifiques et d'un enregistrement de l'EGM correspondant sur le cycle concerné ayant déclenché la commutation.

Selon un deuxième aspect de l'invention, il est proposé d'aménager la commutation automatique de mode de manière à éviter dans un certain nombre de cas le basculement en mode DDD lorsque ce mode ne se justifie pas.

Le premier cas, correspondant au chronogramme illustré sur la figure 1, est celui dans lequel la détection en fenêtre de sécurité (événement référencé "r", par opposition aux événements "R" détectés hors fenêtres de sécurité) est précédée d'un raccourcissement du délai AR sur un certain nombre de cycles précédents, ceci en présence d'un rythme auriculaire stable.

On voit ainsi sur la figure 1 que l'intervalle AR prend les valeurs successives décroissantes 171, 156 ... 109, 93 ms, de sorte que le dernier événement r, dont le délai AR est de 93 ms, survient à l'intérieur de la fenêtre de sécurité de 100 ms.

Dans une telle situation, l'algorithme suspecte une insuffisance chronotrope avec accélération ventriculaire, et inhibe le basculement vers le mode DDD, qui autrement sursit été déclenché avec un dispositif antérieur.

Un autre cas de figure est celui illustré figure 2, avec, inversement, un allongement progressif du délai AR, tel que ce délai devient supérieur à l'intervalle de couplage auriculaire (intervalle AA) et finit par tomber dans la fenêtre de sécurité de la stimulation auriculaire suivante.

Plus précisément, le dispositif considère que l'on se trouve en présence d'une telle situation si la détection r en fenêtre de sécurité est précédée d'un certain nombre de cycles (pas nécessairement consécutifs) pour lesquels le rapport délai AR/délai RR est supérieur par exemple à 75% (l'analyse peut être également effectuée sur le rapport délai AR/délai AA).

Selon un autre aspect de l'invention, il est proposé de modifier l'algorithme de commande du dispositif d'une manière permettant de faire réapparaître les détections ventriculaires survenant en fenêtre de sécurité.

Cette modification vise à éviter les commutations sur des événements de ce type, en essayant de démasquer une éventuelle activité ventriculaire.

Ainsi, sur toute stimulation auriculaire suivie d'une détection en fenêtre de sécurité, il est notamment possible de :
- raccourcir l'intervalle d'échappement auriculaire d'un délai paramétrable, ou
- allonger l'intervalle d'échappement auriculaire d'un délai paramétrable, ou
- suspendre la stimulation auriculaire.

Si l'une de ces modifications révèle une activité ventriculaire démasquée, alors la stimulation auriculaire correspondante n'est pas considérée comme associée à un BAV.

## Revendications

1. Un dispositif médical implantable actif, notamment un stimulateur cardiaque, défibrillateur et/ou cardioverteur, comprenant :
- des moyens de stimulation ventriculaire et auriculaire,
- des moyens de détection d'événements ventriculaires (R, r), opérant avec application d'une fenêtre de sécurité suivant la délivrance d'une impulsion de stimulation auriculaire,
- des moyens aptes à faire fonctionner le dispositif en mode AAI avec détection ventriculaire, ou en mode DDD,
- des moyens de diagnostic de trouble de conduction auriculo-ventriculaire, aptes à déterminer l'apparition d'un bloc auriculo-ventriculaire à partir d'une séquence d'événements auriculaires (A) non suivis, pendant la durée d'un intervalle d'échappement auriculaire, d'une détection d'événement ventriculaire (R) correspondante hors fenêtre de sécurité, et
- des moyens de commutation de mode, aptes à commander conditionnellement le basculement du mode AAI en mode DDD en cas d'apparition d'un bloc auriculo-ventriculaire détecté par les moyens de diagnostic, et
- des moyens discriminateurs d'événements ventriculaires, aptes à détecter la survenue d'au moins un événement ventriculaire (r) en fenêtre de sécurité en l'absence de bloc auriculo-ventriculaire détecté par les moyens de diagnostic, et
dispositif **caractérisé en ce que** les moyens discriminateurs d'événements ventriculaires sont des moyens aptes à inhiber le basculement du mode AAI en mode DDD en cas de détection :
- d'un raccourcissement du délai auriculo-ventriculaire sur un premier nombre prédéterminé de cycles précédant la détection dudit événement ventriculaire (r) en fenêtre de sécurité, concurremment avec un rythme auriculaire stable, ou
- d'un deuxième nombre prédéterminé de cycles pour lesquels le délai auriculo-ventriculaire est supérieur d'une durée ou d'une proportion donnée à l'intervalle de couplage auriculaire.

2. Le dispositif de la revendication 1, dans lequel les moyens discriminateurs d'événements ventriculaires comprennent des moyens pour, en cas de basculement du mode AAI en mode DDD, positionner et associer un premier marqueur audit basculement de mode, ce premier marqueur étant représentatif d'une commutation non liée à un bloc auriculo-ventriculaire.

3. Le dispositif de la revendication 2, dans lequel :
- les moyens de commutation de mode comprennent des moyens pour, après basculement du mode AAI en mode DDD, maintenir le dispositif en mode DDD pendant une durée d'épisode prédéterminée, et
- les moyens discriminateurs d'événements ventriculaires comprennent également des moyens pour positionner et associer un deuxième marqueur à l'épisode ayant débuté sur le basculement associé au premier marqueur, ce deuxième marqueur étant représentatif d'un épisode non lié à un bloc auriculo-ventriculaire.

4. Le dispositif de la revendication 2 ou 3, comprenant des moyens de comptage des premiers et/ou deuxièmes marqueurs.

5. Le dispositif de la revendication 1, dans lequel les moyens discriminateurs d'événements ventriculaires comprennent également des moyens pour modifier la durée de l'intervalle d'échappement auriculaire après détection dudit événement ventriculaire en fenêtre de sécurité.

6. Le dispositif de la revendication 1, dans lequel les moyens discriminateurs d'événements ventriculaires comprennent également des moyens pour suspendre la stimulation auriculaire après détection dudit événement ventriculaire en fenêtre de sécurité.

## Claims

1. An active implantable medical device, in particular a cardiac pacemaker, defibrillator and/or cardioverter, comprising:
- means for stimulating the ventricle and atrium,
- means for detecting ventricular events (R, r), operating with the application of a safety window following the delivery of an atrial stimulation pulse,
- means able to operate the device in AAI mode with ventricular detection or in DDD mode,
- means for diagnosing an atrio-ventricular conduction disorder, able to determine the appearance of an atrio-ventricular block based on a sequence of atrial events (A) not followed, during the duration of an atrial escape interval, by a corresponding detection of a ventricular event (R) outside the safety window, and
- means for mode commutation, able to conditionally command the switching from AAI to DDD mode in case of appearance of an atrio-ventricular block detected by the diagnosing means, and
- means for discriminating ventricular events, able to detect the occurrence of at least one ventricular event (r) inside the safety window in the absence of an atrio-ventricular block detected by the diagnosing means, and
the device being **characterised in that** the means for discriminating ventricular events are means able to inhibit the switching from AAI to DDD mode in case of detection:
- of a shortening of the atrio-ventricular delay over a first predetermined number of cycles preceding the detection of said ventricular event (r) inside the safety window, concurrently with a stable atrial rhythm, or
- of a second predetermined number of cycles for which the atrio-ventricular delay is longer than the atrial coupling interval by a given duration or proportion.

2. The device of claim 1, wherein the means for the discrimination of ventricular events comprise means to, in case of a switching from AAI to DDD mode, position and associate a first marker to said mode switching, this first marker being representative of a commutation not related to an atrio-ventricular block.

3. The device of claim 2, wherein:
- the means for mode commutation comprise means for maintaining the device in DDD mode during a predetermined episode duration after switching from AAI to DDD mode, and
- the means for the discrimination of ventricular events also comprise means for positioning and associating a second marker to the episode that has begun with the switching associated to the first marker, this second marker being representative of an episode that is not related to an atrio-ventricular block.

4. The device of claim 2 or 3, comprising means for counting the first and/or second markers.

5. The device of claim 1, wherein the means for discriminating ventricular events also comprise means for modifying the duration of the atrial escape interval after the detection of said ventricular event inside the safety window.

6. The device of claim 1, wherein the means for discriminating ventricular events also comprise means for suspending atrial stimulation after the detection of said ventricular event inside the safety window.

## Patentansprüche

1. Aktive implantierbare medizinische Vorrichtung, insbesondere Herzschrittmacher, Defibrillator und/oder Kardioverter, mit:
- Mitteln zur Herzkammer- und Herzvorhofstimulation,
- Mitteln zur Erfassung von Herzkammereignissen (R, r), die unter Anwendung eines Sicherheitsfensters im Anschluss an die Abgabe eines atrialen Stimulationsimpulses wirken,
- Mitteln, die geeignet sind, die Vorrichtung im AAI-Modus mit Herzkammererfassung oder im DDD-Modus zu betreiben,
- Mitteln zur Diagnose einer Störung der atrioventrikulären Leitung, die geeignet sind, ausgehend von einer Sequenz von Herzvorhofereignissen (A), die während der Dauer eines atrialen Escape-Intervalls nicht von einer entsprechenden Erfassung eines Herzkammerereignisses (R) außerhalb des Sicherheitsfensters gefolgt werden, das Auftreten eines atrioventrikulären Blocks festzustellen, und
- Mitteln zur Modusumschaltung, die geeignet sind, im Falle des Auftretens eines durch die Diagnosemittel erfassten atrioventrikulären Blocks bedingungsabhängig den Wechsel vom AAI-Modus zum DDD-Modus zu befehlen, und
- Mitteln zur Unterscheidung von Herzkammerereignissen, die geeignet sind, das Auftreten wenigstens eines Herzkammerereignisses (r) innerhalb des Sicherheitsfensters in Abwesenheit eines durch die Diagnosemittel erfassten atrioventrikulären Blocks zu erfassen, und
Vorrichtung, die **dadurch gekennzeichnet ist, dass** die Mittel zur Unterscheidung von Herzkammerereignissen Mittel sind, die geeignet sind, den Wechsel vom AAl-Modus in den DDD-Modus zu unterdrücken, im Falle der Erfassung:
- einer Verkürzung der atrioventrikulären Verzögerung über eine erste vorbestimmte Anzahl von Zyklen, die der Erfassung des Herzkammerereignisses (r) innerhalb des Sicherheitsfensters vorangehen, gleichzeitig mit einem stabilen Herzvorhofrhythmus, oder
- einer zweiten vorbestimmten Anzahl von Zyklen, bei welchen die atrioventrikuläre Verzögerung um eine gegebene Dauer oder Proportion größer als das atriale Kopplungsintervall ist.

2. Vorrichtung nach Anspruch 1, bei welcher die Mittel zur Unterscheidung von Herzkammerereignissen Mittel umfassen, um im Falle eines Wechsels vom AAI-Modus zum DDD-Modus einen ersten Marker zu positionieren und dem Moduswechsel zuzuordnen, wobei dieser erste Marker für eine Umschaltung repräsentativ ist, die nicht mit einem atrioventrikulären Block zusammenhängt.

3. Vorrichtung nach Anspruch 2, bei welcher:
- die Mittel zur Modusumschaltung Mittel umfassen, um nach dem Wechsel vom AAI-Modus zum DDD-Modus die Vorrichtung während einer vorbestimmten Episodendauer im DDD-Modus zu halten, und
- die Mittel zur Unterscheidung von Herzkammerereignissen ebenfalls Mittel umfassen, um einen zweiten Marker zu positionieren und der Episode zuzuordnen, die mit dem dem ersten Marker zugeordneten Wechsel begonnen hat, wobei dieser zweite Marker für eine Episode repräsentativ ist, die nicht mit einem atrioventrikulären Block zusammenhängt.

4. Vorrichtung nach Anspruch 2 oder 3, mit Mitteln zum Zählen der ersten und/oder zweiten Marker.

5. Vorrichtung nach Anspruch 1, bei welcher die Mittel zur Unterscheidung von Herzkammerereignissen ebenfalls Mittel umfassen, um die Dauer des atrialen Escape-Intervalls nach Erfassung des Herzkammerereignisses innerhalb des Sicherheitsfensters zu modifizieren.

6. Vorrichtung nach Anspruch 1, bei welcher die Mittel zur Unterscheidung von Herzkammerereignissen ebenfalls Mittel umfassen, um nach Erfassung des Herzkammerereignisses innerhalb des Sicherheitsfensters die Herzvorhofstimulation auszusetzen.
